(19) **Europäisches Patentamt**
**European Patent Office**
**Office européen des brevets**

(11) **EP 3 689 251 A1**

(12) **EUROPEAN PATENT APPLICATION**

(43) Date of publication:
05.08.2020 Bulletin 2020/32

(51) Int Cl.:
*A61B 8/12* (2006.01)   *A61B 8/00* (2006.01)
*B06B 1/02* (2006.01)   *G01S 7/52* (2006.01)

(21) Application number: 19154455.0

(22) Date of filing: 30.01.2019

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **VAN RENS, Antonia Cornelia**
**5656 AE Eindhoven (NL)**
• **MOUNAIM, Amine**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **IMPROVED INTERVENTIONAL MEASUREMENTS**

(57)    The present invention relates to interventional measurements. In order to provide improved interventional measurements, a device (10) for interventional measurements is provided. The device comprises a elongate body (12) configured to be at least partly inserted into an anatomical structure of a subject, and a transducer arrangement (14) configured to being energized, and to receive sensing signals and to provide respective signals to a console, and an amplifier circuit (16) configured to amplify the signals from the transducer arrangement; and a wiring arrangement (18) along the elongate body providing: i) electric energy supply for the transducer arrangement and the amplifier circuit; and ii) signal data transmission of the signals from the transducer arrangement. The transducer arrangement and the amplifier circuit are provided in a distal end region of the elongate body. The wiring arrangement comprises a ground and a supply. The transducer arrangement is connected to the supply and to the ground; and the amplifier circuit is connected to the supply, to the transducer arrangement and to the ground. Between the transducer arrangement and the ground, a connection selector (20) is provided that is electrically non-conductive below a threshold current or threshold voltage, and electrically conductive above the threshold current or the threshold voltage.

**FIG. 2**

## Description

FIELD OF THE INVENTION

The present invention relates to a device for interventional measurements, to a console for interventional measurements, to a system for interventional measurements and to a method for interventional measurements.

BACKGROUND OF THE INVENTION

For improvement in healthcare procedures, imaging and sensing devices can be integrated at the tip of interventional instruments, such as catheters and guide wires. Integrating the required imaging/sensing functionality is challenging in view of the size of these instruments. As an example, micro-machining sensors, e.g. provided by micro-electromechanical systems, MEMS, are used and enable advanced imaging and/or sensing functionality in a very small space. For example, US 2013/030919 A1 relates to circuit architectures and electrical interfaces for rotational intravascular ultrasound devices. A rotational intravascular ultrasound comprises a flexible elongate member. A piezoelectric micromachined ultrasound transducer (PMUT) is coupled to a distal portion of the flexible elongate member. A circuit is coupled to the distal portion of the flexible elongate member. The circuit is electrically coupled to the PMUT and includes a pulser for driving the PMUT, an amplifier for receiving and amplifying signals representative of ultrasound echoes received by the PMUT, a protection circuit configured to protect the amplifier from high voltage transmit pulses from the pulser and allow the amplifier to receive the low amplitude echo signals from the PMUT, and timing and control circuitry. To isolate the sensitive amplifier inputs from the high voltage transmit pulses used to excite the transducer, a protection circuit is provided for the amplifier circuit that is attached in close proximity to the transducer in some instances. The protection circuit blocks the high voltage transmit pulse (100 V) from reaching the amplifier inputs, but it allows the low amplitude echo signals (typically 1 V or less) to reach the amplifier inputs with minimal loss attributable to the combination of series resistance and shunt capacitance of the protection circuit. However, it has been shown that there is a need for further miniaturization, lower costs and improved signal quality.

SUMMARY OF THE INVENTION

There is a need to provide improved interventional measurements.

The object of the present invention is solved by the subject-matter of the independent claims; further embodiments are incorporated in the dependent claims. It should be noted that the following described aspects of the invention apply also for the device for interventional measurements, for the console for interventional measurements, for the system for interventional measurements and for the method for interventional measurements.

According to the present invention, a device for interventional measurements is provided. The device comprises a flexible elongate body configured to be at least partly inserted into an anatomical structure of a subject, e.g. in a vessel by steering through the vasculature. The device also comprises a transducer arrangement configured to being energized from an electric energy supply, e.g. for generating measuring emission in the first mode, and to receive sensing signals and to provide respective signals to a console, e.g. to receive sensing waves in a second mode, to transform the sensing waves into signals and to provide respective signals to a console. The device further comprises an amplifier circuit configured to amplify the signals from the transducer arrangement. The device still further comprises a wiring arrangement along the flexible elongate body providing: i) electric energy supply for the transducer arrangement and the amplifier circuit; and ii) signal data transmission of the signals from the transducer arrangement. The transducer arrangement and the amplifier circuit are provided in a distal end region of the flexible elongate body. The wiring arrangement comprises a ground and a supply. The transducer arrangement is connected to the supply and to the ground, and the amplifier circuit is connected to the supply, to the transducer arrangement and to the ground. Between the transducer arrangement and the ground, a connection selector is provided that is electrically non-conductive below a threshold current or threshold voltage, and electrically conductive above the threshold current or the threshold voltage.

This provides a protection for the amplifier circuit. The protection is provided in a simplified setup with reduced complexity requiring only a minimum number of wirings along the shaft member of the interventional device.

In an example, non-conductive is referred to as non-transmitting.

In an example the interventional device is one of an intravascular device (guidewire, catheter, sheath) or a needle (biopsy, treatment).

In an example the transducer is one of a pressure sensor, a flow velocity sensor, an ultrasound imaging sensor. In a further example the transducer is a micromachined transducer from the group piezoelectic and capacitive micromachined transducers.

According to an example, the device is configured as a device for intravascular ultrasound imaging. The transducer arrangement is an ultrasound transducer arrangement configured to emit ultrasound waves in the first mode, and to receive ultrasound echo waves and to provide respective signals in the second mode.

According to an example, the connection selector comprises two antiparallel diodes. The diodes are provided with a diode characteristic matching with a characteristic of at least one of the group of the electric energy supply for the transducer arrangement and the electric energy supply for the amplifier circuit and the characteristics of the amplifier circuit. The voltage and/or current of the electric energy supply for the transducer arrangement is above the threshold current or threshold voltage of the diode characteristic.

According to an example, the ground comprises a ground conductor. The wiring arrangement is i) a two-wire connection comprising only the ground conductor and the supply comprises one supply conductor, or ii) a three-wire connection comprising only the ground conductor and the supply comprises one transducer-supply conductor and one amplifier-supply conductor.

According to the present invention, also a console for intravascular measurements is provided. The console comprises a first electric energy supply for supply of a transducer arrangement. The console also comprises a second electric energy supply for supply of an amplifier circuit. The console further comprises a unit for amplifying a received signal. The console still further comprises an interface arrangement comprising at least one supply interface for connection with a device for intravascular measurements according to one of the preceding examples.

According to an example, a switching arrangement is provided for alternate connection of the first electric energy supply or the amplifier to the supply interface. The second electric energy supply remains connected to the supply interface.

According to an example, the interface arrangement comprises a first supply interface connected to the first electric energy supply. The first supply interface is provided for connection with a transducer supply of the device for intravascular ultrasound imaging. The interface arrangement also comprises a second supply interface connected to the second electric energy supply and the unit for amplifying. The second supply interface is provided for connection with an amplifier circuit of the device for intravascular ultrasound imaging.

According to the present invention, also a system for intravascular measurements is provided. The system comprises a device for intravascular measurements according to one of the examples above. Further, a console according to one of the examples above is provided. For intravascular measurements, the device for intravascular measurements is connectable to the console.

According to the present invention, also a method for intravascular measurements is provided. The method comprises the following steps:

a) providing an electrical stimulus voltage/current to a transducer arrangement, provided in a distal end region of a flexible elongate body configured to be at least partly inserted into an anatomical structure of a subject, e.g. in a vessel by steering through the vasculature, generating measuring emission in a first mode;

b1) receiving sensing waves by the transducer arrangement and providing respective signals in a second mode;

b2) providing an electrical amplification supply voltage/current to an amplifier circuit provided in the distal end region of the flexible elongate body and amplifying the signals from the transducer arrangement with the amplifier circuit; and

c) supplying the amplified signals to a proximal end of the flexible elongate body.

The electrical stimulus voltage/current, the electrical amplification supply voltage/current and the amplified signals are provided by a wiring arrangement along the flexible elongate body, the wiring arrangement comprising a ground and a supply. The transducer arrangement is connected to the supply and to the ground. The amplifier circuit is connected to the supply, to the transducer arrangement and to the ground. The transducer arrangement and the ground are selectively connected with a connection selector that is electrically non-conductive below a threshold current or threshold voltage, and electrically conductive above the threshold current or the threshold voltage.

The electrical amplification supply voltage/current, i.e. step b2), is provided at during at least a part of the duration of the receiving of the sensing waves, i.e. during step b1) when the sensing takes place and the signals should be amplified.

The stimulus voltage/current can also be referred to as sensor supply current.

According to an aspect, the connection selector is provided to address the need for a high supply so-to-speak flowing from the console to the tip of the wire (for generating the emission at the tip), while also having the need to improve weak signals that so-to-speak flow back (transmission) from the tip to the console. In other words, the connection selector allows having a two-way supply.

According to an aspect, a mix of high current and high voltage is provided. The selector (acting as a switch) is placed in series with the transducer. The selector will behave as a high impedance in case of low currents and as a low impedance in case of high currents. The threshold current typically is determined by a linear component, e.g. a resistor that is placed across an operational amplifier (opamp) in a transimpedance configuration, that absorbs the current when below a current threshold and that forces the current into the diodes when above the threshold.

In a first attempt, it is avoided that the amplifier sees high voltage. In a second attempt, the high voltage is provided across the transducer to generate acoustic energy, which is another reason to avoid high voltage across the amplifier.

The function of the connection selector, e.g. the diodes, is to provide a selection: High energy supply (high currents) are passing through the selector to directly feed the transducer and to avoid that the amplifier sees a high voltage (i.e. to avoid that the amplifier has to be high-voltage compliant). HF and DC low energy supply (low currents) are blocked, both for AC and DC as the transducer is high impedant for DC. For AC (alternating current), current starts flowing. Hence, low currents are blocked by the selector to deviate the current flow such that this has to pass the amplifier circuit to make sure it gets amplified. Due to alternating current, in case of diodes, a diode is needed for each direction, i.e. the anti-parallel arrangement. The diodes, and also the operational amplifier and the feedback resistors, are chosen such that the respective threshold value is met. The threshold value is defined by the system requirements and the circuit components are then selected accordingly.

These and other aspects of the present invention will become apparent from and be elucidated with reference to the embodiments described hereinafter.

BRIEF DESCRIPTION OF THE DRAWINGS

Exemplary embodiments of the invention will be described in the following with reference to the following drawings:

Fig. 1 schematically shows a part of an example of a device for intravascular measurements.

Fig. 2 shows an example of a functional diagram for the device of Fig. 1.

Fig. 3 shows another example of a functional diagram for the device of Fig. 1 with a separate energy supply for the transducer arrangement.

Fig. 4 shows an example of a more detailed circuit diagram for the device of Fig. 1.

Fig. 5 shows another example of a more detailed circuit diagram for the device of Fig. 1 with a separate energy supply for the transducer arrangement.

Fig. 6 shows an example of a console for interventional measurements.

Fig. 7 shows an example of a circuit diagram of a console for interventional measurements.

Fig. 8 shows another example of a circuit diagram of a console for interventional measurements with a separate energy supply for the transducer arrangement.

Fig. 9 shows an example of a system for interventional measurements.

Fig. 10 shows basic steps of an example of a method for interventional measurements.

DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

The term "subject" may also be referred to as individual. The "subject" may further also be referred to as patient, although it is noted that this term does not indicate whether any illness or disease is actually present with the subject.

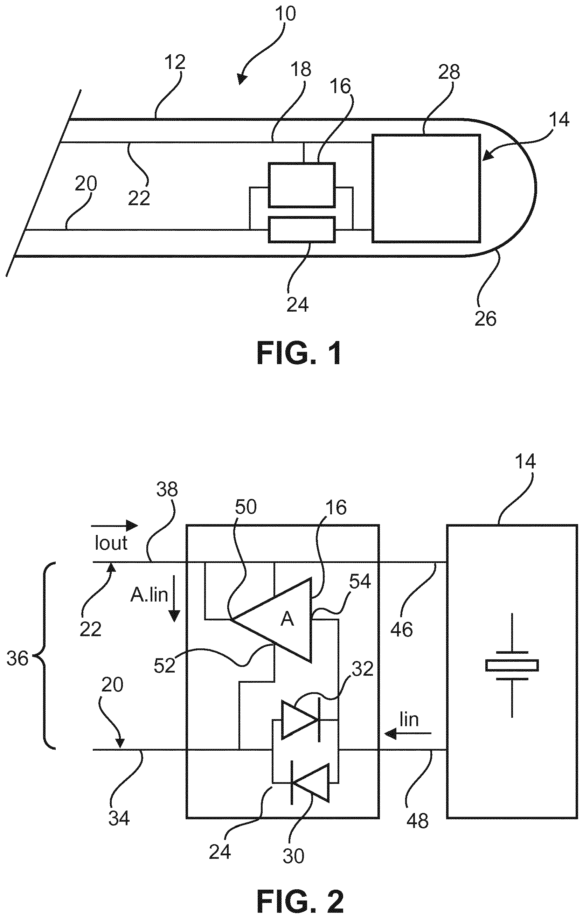

Fig. 1 schematically shows a part of an example of a device 10 for interventional measurements. The device 10 comprises a flexible elongate body 12 configured to be at least partly inserted into an anatomical structure of a subject, e.g. a vessel or an organ. The device 10 also comprises a transducer arrangement 14 configured to being energized by an energy supply source, e.g. to generate measuring emission in a first mode, and to receive sensing signals and to provide respective signals to a console, e.g. in a second mode.

In an exemplary embodiment the transducer arrangement 14 is configured to generate measuring emission in a first mode, and in a second mode to provide measurement signals from the transducer arrangement to the console.

The device 10 further comprises an amplifier circuit 16 configured to amplify the signals from the transducer arrangement. The device 10 still further comprises a wiring arrangement 18 along the flexible elongate body providing: i) electric energy supply for the transducer arrangement and the amplifier circuit; and ii) signal data transmission of the signals from the transducer arrangement. The transducer arrangement and the amplifier circuit are provided in a distal end region of the flexible elongate body. The wiring arrangement comprises a ground 20 and a supply 22. The transducer arrangement is connected to the supply and to the ground; and the amplifier circuit is connected to the supply, to the transducer arrangement and to the ground. Between the transducer arrangement and the ground, a connection selector 24 is provided that is electrically non-conductive below a threshold current or threshold voltage, and electrically conductive above the threshold current or the threshold voltage.

A rounded distal end portion 26, i.e. a tip portion, indicates the suitability for insertion into a vascular structure and steering the device therein.

The term "flexible elongate body" relates to a structural component that is suitable for being inserted and guided within the vascular structure of the subject. The flexible elongate body is hence stable enough to allow a forward pushing movement and also flexible enough to follow curved vascular structures. Further, means are provided to allow a guiding of the tip or front region. The flexible elongate body can also be referred to as guidewire.

The term "distal end" relates to the end away from the user steering the device. The distal end is the end of the body that is inserted first and that forms the tip when steering forward in the vessels.

The term "transducer arrangement" relates to one or more components that provide both functions of energizing a transducer element which will then trigger or generate respective measurement signals.

In an example energizing a transducer element comprsies the function of generating an emission that will then result in back reflected or echoed or mirrored waves, i.e. emissions in view of the arrangement, which will then trigger or generate respective signals. The transducer arrangement is thus bi-functional.

The term "transducer" relates to energizing a transducer element (or in other words a sensor) for measuring parameters of anatomical structures (e.g. blood, vessel wall, tissue) surrounding the transducer arrangement, in particular in the distal portion of the device. The parameter may be physiological parameter such as pressure, flow velocity, temperature, electrical activation signal of an anatomy, it can be a parameter derived from the appearance of the anatomy such as geometry, type of tissue.

In an exemplary embodiment the transducer relates to generating acoustic energy from electric energy and vice versa. The transducer is thus a two-way component.

The transducer is also used for receiving for sensing purposes. When sensing, there is one scenario where the transducer emits and receives. However, another scenario is also possible, where the transducer only receives, and the emission waves are generated by another element.

The transducer arrangement can also be referred to as bi-functional sensor arrangement, or as active sensor arrangement. It is noted that the transducer arrangement can also be used for further purposes, for example in an active supply mode when generating emission used for e.g. therapeutic purposes. For example, the emission of energy has a purpose to perform a therapy, like high-intensity focused ultrasound (HIFU) application. In an option, the arrangement is then also used to sense as well, e.g. to get some feedback info on the therapy. An example can be to monitor the progress of a therapy.

The term "amplifier circuit" relates to an arrangement of electrical components and elements provided in a circuity arrangement that provides an amplification of the signal of the sensor.

The term "wiring arrangement" relates to the provision of electric conductors on the main shaft or main body. The wiring arrangement thus electrically connects the transducer arrangement and the amplifier circuit with a base or console. The wiring arrangement can thus relate to electric tracks on or within the shaft or wires arranged on or within the shaft.

The connection selector can also be referred to as connectivity selector.

The connection selector in combination with the usage for resistors in the amplifier circuit allows the use of low voltage transistors for the amplifier circuit. The amplifier circuit may comprise MOS transistors.

If the current is low enough, all current will flow to the amplifier circuit. If the current is larger than the threshold, the voltage swing across the transducer is very high (polarity can be positive or negative), and hence the connection selector is letting the current through.

The wire arrangement this comprises only a very limited amount of wires. This provides spatial and constructive advantages for the connection between the sensors and a readout system. The minimized number of wires, i.e. the small or low wire-count thus only requires a limited space for wire routing. Further, the low wire-count also simplifies soldering and bonding of the system wires to sensor die(s).

In an example, the device 10 is configured as a device for intravascular ultrasound imaging, wherein the transducer arrangement is an ultrasound transducer arrangement 28 configured to emit ultrasound waves in the first mode, and to receive ultrasound echo waves and to provide respective signals in the second mode.

The device for intravascular ultrasound imaging can also be referred to as intravascular ultrasound device.

In the first mode, in which the ultrasound transducer arrangement emits ultrasound waves, the connection selector is providing a transmit mode. In the second mode, in which the ultrasound transducer arrangement receives ultrasound echo waves and provides respective signals, the connection selector is providing a receive mode.

In an example, the connection selector 20 is provided as a passive component with a connection selection based on a present electrical current.

The passive component is not requiring any triggering or activating signals.

The connection selector is provided as a switchless or switch-free circuit.

Fig. 2 shows an example of a functional circuit diagram for the device 10 of Fig. 1. The transducer arrangement 14 is shown on the right in a first frame. The amplifier circuit 16, also indicated with A, is shown left in a second frame. The connection selector 24 is arranged below the amplifier circuit 16 in the second frame.

As an option, the connection selector 24 comprises two antiparallel diodes 30, 32. The diodes 30, 32 are provided with a diode characteristic matching with a characteristic of at least one of the group of the electric energy supply for the transducer arrangement 14 and the electric energy supply for the amplifier circuit 16 and the amplifier characteristics. The voltage and/or current of the electric energy supply for the transducer arrangement 14 is above the threshold current or threshold voltage of the diode characteristic.

The term "anti-parallel" relates to an arrangement where the diodes are connected in parallel but with opposite orientation concerning the direction of their blocking effect.

In an example, the transducer arrangement 14 is supplied with a unipolar or bipolar stimulus voltage, e.g. with AC current.

In an example, the transducer arrangement, e.g. the micromachined transducer, is supplied with bipolar high voltage. The micromachined transducer may

The term "high voltage" refers to a voltage in the range of about 10 to 100 Volt, for example above 30 Volt

The term "low voltage" refers to a voltage in the range of about 1.2 to 3.6 Volt, for example below 2.5 Volt.

For example, an AC voltage of 24 Volt is provided, i.e. the connection selector is subject to ±24 Volt. The two diodes provide threshold function to conduct current for both directions of the AC current supply.

The ground 20 comprises a ground conductor 34.

In a first option, shown in Fig. 2, the wiring arrangement 18 is a two-wire connection 36 comprising only the ground conductor 34 and the supply comprises one supply conductor 38.

The supply and the amplified signal output are conducted via the same wire, i.e. the supply wire. The amplified signal is summed to the supply for the amplifier circuit.

A supply from an energy supply is indicated with $I_{out}$ and a supply for the amplifier circuit is indicated with $A.I_{in}$.

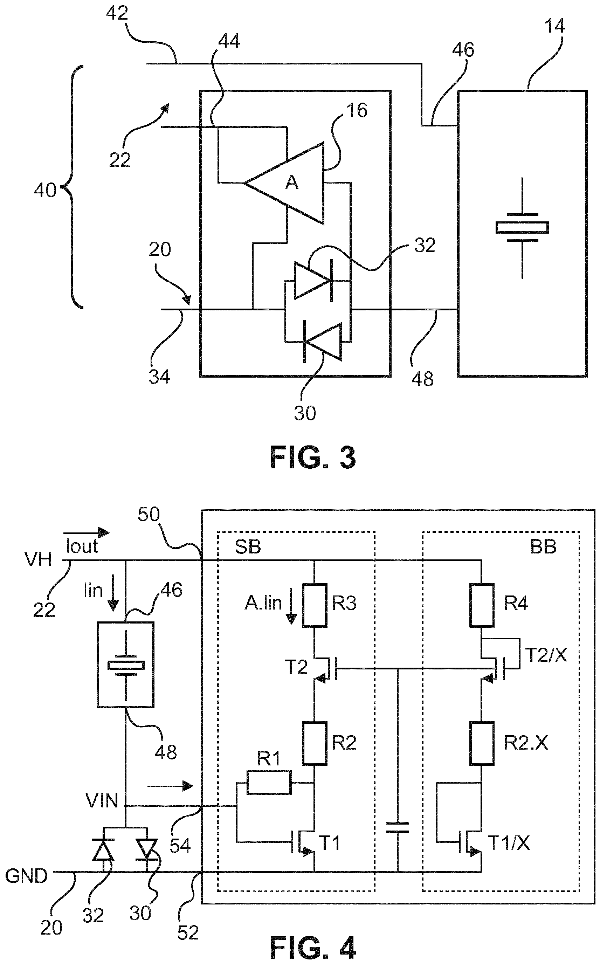

Fig. 3 shows another example of a functional circuit diagram for the device of Fig. 1 with a separate energy supply for the transducer arrangement.

In a second option, shown in Fig. 3, the wiring arrangement 18 is a three-wire connection 40 comprising only the ground conductor 34 and the supply comprises one transducer-supply conductor 42 and one amplifier-supply conductor 44. Tracks on a composite-core wire (not shown) are connected It also is possible to use normal thin wires between a first node *VH* and a second node *GND.*

The transducer arrangement 14 is connected with a first transducer-port 46 (also referred to as transducer terminal) to the supply, and with a second transducer-port 48 to the ground 20.

The amplifier circuit 16 has three terminals and is connected with a first amplifier-port 50 (also referred to as amplifier terminal) to the supply 22, i.e. the first node *VH,* and with a second amplifier-port 52 to the ground 20, i.e. the second node *GND,* and with a third amplifier-port 54, i.e. with *VIN,* to the second transducer-port 48. The first amplifier-port 50 serves as ASIC supply. The first amplifier terminal also connects to the first transducer terminal. The connection selector 24 is arranged between the second transducer-port 48 and the third amplifier-port 54 and the ground 20.

Fig. 4 shows an example of a more detailed circuit diagram for the device of Fig. 1. In this example, the amplifier circuit 16 comprises low voltage transistors *T*, wherein resistors R are arranged between the supply 22 and the transistors T. The resistors R absorb the overdrive in voltage such that the voltage across the transistors T remains within the normal low voltage range for all supply voltages.

A connection to a supply, also supply high voltage, is indicated with *VH.* Ground is indicated with *GND.* An input of the connection selector is indicated with *VIN.*

Fig. 4 shows a mode when signals from the transducer arrangement are getting amplified by the amplifier circuit.

The amplifier circuit may be provided with a signal branch *SB* and a bias branch *BB.*

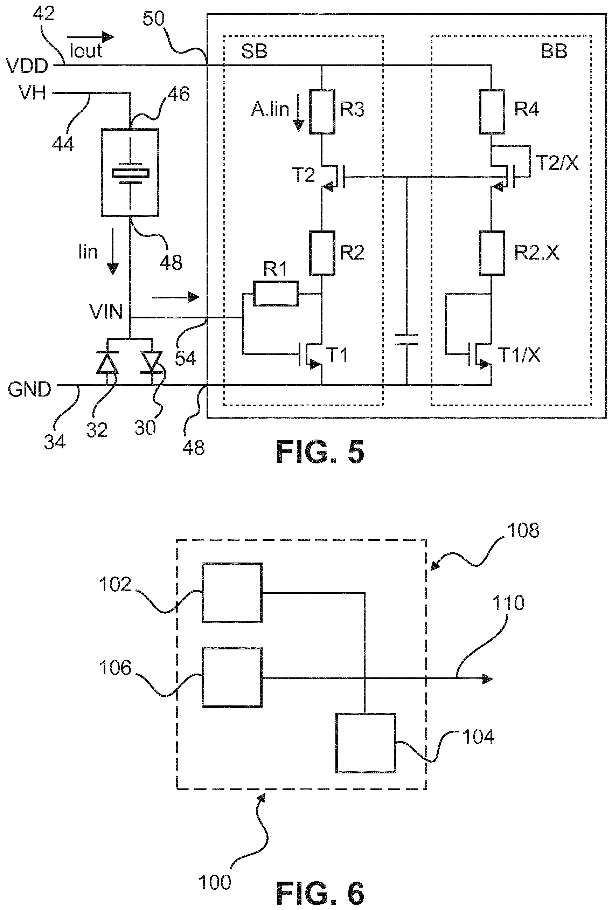

Fig. 5 shows another example of a more detailed circuit diagram for the device of Fig. 1 with a separate energy supply for the transducer arrangement.

In further examples, other measures to protect the amplifier circuit are provided, e.g. blocking, protecting, covering and/or absorbing the high voltage bipolar supply voltage for the transducers.

The device for intravascular ultrasound imaging is provided as a flow-wire. In an example, the flow-wire is based on Doppler ultrasound and uses a conventional ultrasound transducer element, which is placed forward looking in the guidewire. The amplified sensor signals, i.e. the returning sensor signals, improve for example the electrical signal characteristics, in particular for high-frequency signals. As an example, this makes the device suitable to be provided as a composite-core wire which provides benefits in terms of deliverability, i.e. steerability by the user, and also costs.

The circuit amplifies the weak transducer echo signals in receive mode and is transparent in transmit mode. The amplification in RX mode improves the signal strength and the noise factor as well as the signal integrity.

The device for intravascular ultrasound imaging with only two wires along the wire shaft is suitable for the use with piezo- and capacitive transducers. As an option, in case of capacitive transducers (CMUT), one additional wire can be provided.

In an example, the device for intravascular ultrasound imaging is provided in an ultrasound Doppler-flow catheter or guidewire.

In further examples, the device is provided with various other sensor types. As an example, the sensor is a high-impedance sensor that is connected directly to low-impedance system wires in catheters of guidewires.

In an example, the transducer arrangement is configured to be supplied by a bipolar energy supply with positive and negative voltage pulses.

Fig. 6 shows an example of a console 100 for interventional measurements. The console 100 comprises a first electric energy supply 102 for supply of a transducer arrangement. The console 100 also comprises a second electric energy supply 104 for supply of an amplifier circuit. The console 100 also comprises a unit 106 for amplifying a received signal. The console 100 further comprises an interface arrangement 108 comprising at least one supply interface 110 for connection with a device 10 for interventional measurements according to one of the preceding examples.

The console can also be referred to as readout unit or connection unit.

In an example the console is for intravascular ultrasound imaging, the console comprising the first electric energy supply for supply of an ultrasound transducer arrangement. The interface arrangement comprises at least one supply interface for connection with a device for intravascular ultrasound imaging according to one of the preceding examples.

In an example, the first electric energy supply comprises a high voltage transmitter circuit.

In an example, the second electric energy supply provides a supply voltage of e.g. approximately 3 Volt direct voltage.

The second electric energy supply remains on also during the supply of the transducers by the first electric energy supply.

In the sensing mode, the bias current of the amplifier supply will be added by variation due to the signal provided in an amplified form.

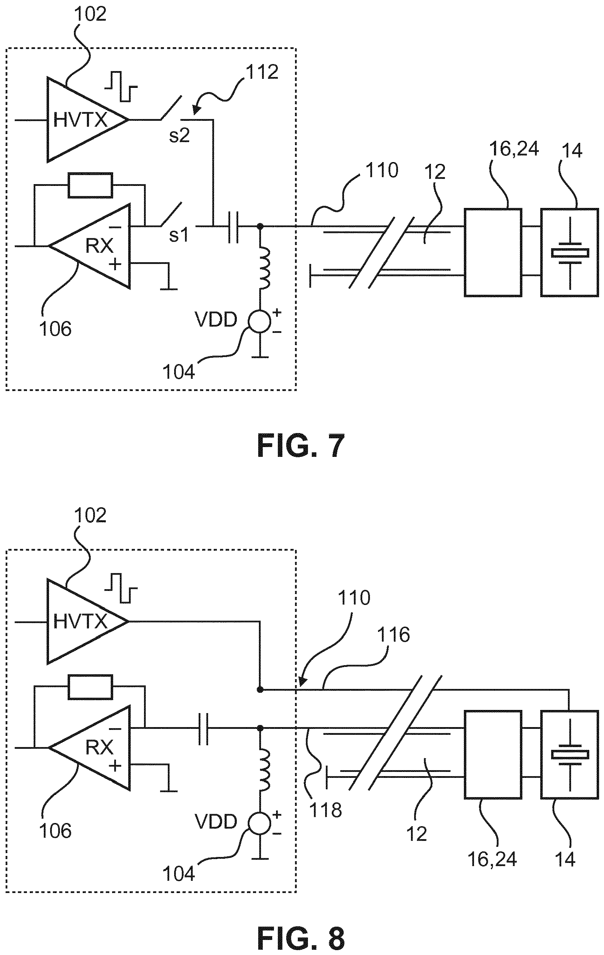

Fig. 7 shows an example of a circuit diagram of a console for interventional measurements. In an example, a switching arrangement 112 is provided for alternate connection of the first electric energy supply 102 or the amplifier 106 to the supply interface 110. The second electric energy supply 104, also indicated with *VDD,* remains connected to the supply interface 110.

A first switch *S1* connects the amplifier 106, also indicated with *RX,* to the supply interface 110, and a second switch *S2* connects the first electric energy supply 102, also indicated with *HV TX,* to the supply interface 110. The first electric energy supply 102 provides an alternating voltage/current (or alternating signal).

The amplifier 106 has a reference connection 114 to ground (HVTX also has a connection to ground).

Fig. 8 shows another example of a circuit diagram of a console for interventional measurements with a separate energy supply for the transducer arrangement. The interface arrangement 108 comprises a first supply interface 116 connected to the first electric energy supply 102, the first supply interface for connection with a transducer supply of the device for intravascular ultrasound imaging. The interface arrangement 108 also comprises a second supply interface 118 connected to the second electric energy supply 104 and the unit 106 for amplifying. The second supply interface 118 is provided for connection with an amplifier circuit of the device for intravascular ultrasound imaging.

Switches are not needed for this example. A combination of high voltage and low voltage is avoided.

In an example, the transducer arrangement is supplied by a bi-polar energy supply with positive and negative voltages.

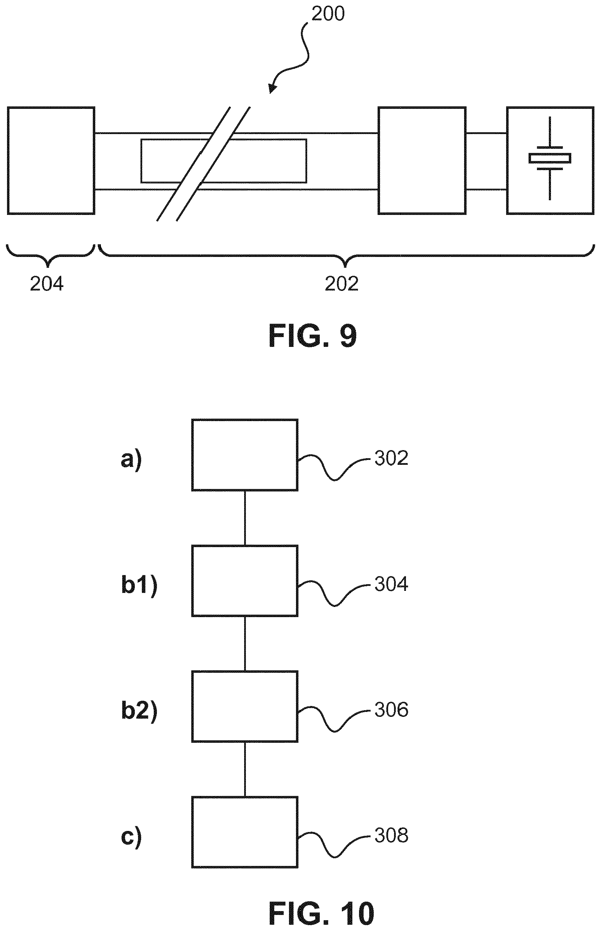

Fig. 9 shows an example of a system 200 for interventional measurements. The system 200 comprises a device 202 for interventional measurements according to one of the examples above and a console 204 according to one of the examples above. The device for interventional measurements is connectable to the console.

Fig. 10 shows basic steps of an example of a method 300 for interventional measurements. The method 300 comprises the following steps:

- In a first step 302, also referred to as step a), in a first mode, electrical stimulus voltage/current is provided to a transducer arrangement arranged in a distal region of a flexible elongate body configured to be at least partly inserted into an anatomical structure of a subject, and the transducer arrangement is energized, e.g. measuring emission is generated.
- In a second step 304, also referred to as step b1), in a second mode, sensing waves are received by the transducer arrangement and respective signals are provided.
- In a third step 306, also referred to as step b2), an electrical amplification supply voltage/current is provided to an amplifier circuit provided in the distal end region of the flexible elongate body and amplifying the signals from the transducer arrangement with the amplifier circuit; and
- In a fourth step, also referred to as step c), the amplified signals are supplied to a proximal end of the flexible elongate body.

The electrical stimulus voltage/current, the electrical amplification supply voltage/current and the amplified signals are provided by a wiring arrangement along the flexible elongate body, the wiring arrangement comprising a ground and a supply. The transducer arrangement is connected to the supply and to the ground. The amplifier circuit is connected to the supply, to the transducer arrangement and to the ground. The transducer arrangement and the ground are selectively connected with a connection selector that is electrically non-conductive below a threshold current or threshold voltage, and electrically conductive above the threshold current or the threshold voltage.

During receive mode, the application-specific integrated circuit (i.e. the ASIC amplifier) senses the weak transducer currents $I_{in}$ and generates an amplified version $A.I_{in}$. The sum of the two currents $I_{out}$ flows via the composite-core wires (also referred to as tracks) to the system electronics circuit for further signal processing.

The same tracks also provide the supply voltage for the amplifier circuit. During transmit, bi-polar high voltage *HV* pulses are provided to drive the transducer. The ASIC routes the pertaining currents to the ground node via a set of anti-parallel diodes. The ASIC amplifier circuit behaves as a relative high impedance load in during this phase.

In an option, a CMUT transducer is provided. Such transducer may need a bias voltage of e.g. 40 Volts (could be 100 V as well) for proper operation. The need for a high bias voltage leads to increased dissipation in the amplifier circuit. As an option, one more node is added to the circuit interface. An extra track, i.e. wire, is provided in the composite-core as an option.

An example of an implementation of the proposed ASIC amplifier circuit is shown in Fig. 4. The circuit is meant to show the functionality only. In further examples, different implementations are provided that serve the same purpose, i.e. provided the same functionality.

The transducer operates in two modes. In a first mode, the transducer is energized, e.g. emission of ultrasound waves are generated. In a second mode, sensing waves are received and respective signals are provided.

In view of the function of the connection selector and the amplifier, the first mode is also referred to as transmit mode and the second mode is referred to as receive mode.

When receiving weak echo signals from the transducer, the anti-parallel diodes have no function. An input transistor $T1$ of the circuit is biased such that the parallel diodes are off. The voltage on $VH$ is stable, for example +3 V and serves as the supply voltage for the amplifier circuit. In this mode, the amplifier senses transducer current $I_{in}$ and amplifies it to $A.I_{in}$, with $A$ the current gain of the circuit. The value of $A$ primarily is determined by the equation below.

$$A=(R(R1))/(R(R2)+1/gm\_T2)$$

In this formula, $R(R1)$ and $R(R2)$ are resistor values of $R1$ and $R2$ and $gm\_T2$ is the trans-conductance of transistor $T2$. For example, $A$ could be 10. The sum of the transducer current and the amplified current will flow via the composite-core wire tracks $VH$ and $GND$.

For robust circuit biasing, in an example, a bias-branch is provided in the amplifier circuit which implements identical (but scaled) components as the signal branch. The current of the bias-branch primarily is defined by the value of a high-impedance resistor $R4$ in combination with supply voltage $VH$. The bias-branch defines the gate-voltage of transistor $T2$, hereby defining the bias-currents in the signal branch. In this example, the current in the signal branch is X times the current in the bias-branch.

The construction of transducer and amplifier circuit with combined supply node forms a feed forward loop. For stability, the overall loop gain ($ALOOP$) must be < 1 for all frequencies. The loop gain is highest around the transducer resonance frequency and can be estimated by:

$$A\_LOOP=((R\_TR//Z\_CHAR))/R\_TR.A$$

In this equation, A is the gain of the amplifier, $RTR$ is the real part of the transducer impedance and $ZCHAR$ is the characteristic impedance of the connected wires. A typical transducer may have an impedance of 1 k$\Omega$ and the characteristic impedance of the composite wire may be 50 $\Omega$.

When ultrasound waves are generated, bi-polar (or uni-polar) high-voltage transmit pulses may be fired to drive the transducer. The resulting high currents are sinked (sunk) to, or sourced from the $GND$ node via the anti-parallel diodes at the input of the ASIC. In this mode, the amplifier circuit does not have a real purpose; it should survive the high voltages and it should not draw too much current. To achieve both purposes, as an example, resistors $R3$ and $R4$ form the interface between the high voltage node "$HV$" and the active transistors on the ASIC. Standard resistors are capable of handling high voltages and the resistor values are chosen such that the resulting circuit currents are acceptable. Further improvement is possible by designing bias circuitry that has a low sensitivity to supply variations, meaning that the current will not grow too much during $HV$ pulses.

Special attention must be paid to the behavior of the circuit when firing negative voltage pulses. Parasitic junction diodes and transistors can start conducting and this may lead to undefined current paths and high current levels; even latch-up may happen. By choosing sufficiently high-impedance values for $R3$ and $R4$ and by placing sealrings (highly doped grounded P+ / N+ rings) around transistor $T2$ and $T2/X$, these risks can be minimized. Sealrings can also be placed around the anti-parallel diode structures.

In an example, transistors $T2$ and $T2/X$ are $LV$ (low-voltage) or $HV$ (high-voltage) type of transistors. Usage of $LV$ transistors provides higher trans-conductance (and therefore gain) and reduced area and parasitic capacitance. When using $LV$ transistors, the voltage on the drain of transistor $T2$ must be limited, for example by a stack of forward biased diodes between the drain of $T2$ and $GND$. Note that the proposed amplifier circuit can be build using standard low-voltage ASIC technology; no high-voltage ASIC technology is needed. This is attractive from cost point of view as well as from silicon real-estate point of view.

The amplifier circuit ASIC and the transducer are connected via the composite-core tracks to a system interface circuit.

In an example, the system interface circuit consists of a $HV$ transmitter circuit that is connected via first switch and a $LV$ trans-impedance amplifier that is connected via a second switch. During operation, the first switch or the second switch conduct. The first switch conducts in the transmit mode and the second switch conducts in the receive mode. The ASIC supply voltage is provided by a voltage source which is connected to the *VH* track via a high-value inductor.

As indicated above, in an option, a capacitive micromachined transducer (CMUT) is provided for the transducer. A supply a bias voltage, e.g. + 40 Volts, for proper operation, provides need for a high bias voltage which leads to increased dissipation in the amplifier circuit. To limit dissipation, in an example, an extra wire is added to the circuit interface, as shown in Fig. 5.

The ASIC circuit implementation can be simplified in this case as the circuit operates with a dedicated supply and readout voltage *VDD.* Concerns on instability, circuit breakdown and latchup have diminished. Node *VH* will only provide the CMUT bias voltage and the *HV* stimulus pulses.

In addition, also the system interface circuit can be simplified. The switches *S1* and *S2* are not provided in an example and the transducer control voltage *VH* and the supply and readout voltage *VDD* are connected via dedicated tracks on the composite core-wire.

In any of the exemplary embodiments the transducer may be one of a pressure sensor, a flow velocity sensor, an ultrasound imaging sensor. The transducer may be a micromachined transducer from the group piezoelectic and capacitive micromachined transducers. In any of the examples the interventional device may be one of an intravascular device (guidewire, catheter, sheath) or a needle (biopsy, treatment). Advantage of the piezoelectric micromachined transducer (PMUT) or the capacitive micromachined transducer (CMUT) is that it can be used for measurement of pressure of blood in the vessels and ultrasound imaging, in alternativ or simultaneous functionality. From ultrasound imaging flow velocity of the blood in the vessel can be derived. In consequence, any of the PMUT or CMUT transducer can also measure blood pressure and blood flow velocity in alternative or simultaneous measurement mode.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A device (10) for interventional measurements, the device comprising:

   - an elongate body (12) configured to be at least partly inserted into an anatomical structure of a subject;
   - a transducer arrangement (14) configured to be energized through an electric energy supply, to receive sensing signals and to provide respective signals to a console;
   - an amplifier circuit (16) configured to amplify the signals from the transducer arrangement; and
   - a wiring arrangement (18) along the elongate body providing: i) electric energy supply for the transducer arrangement and the amplifier circuit; and ii) signal transmission of the sensing signals from the transducer arrangement;

   wherein the transducer arrangement and the amplifier circuit are provided in a distal portion of the elongate body;
   wherein the wiring arrangement comprises a ground and a supply;
   wherein the transducer arrangement is connected to the supply and to the ground; and the amplifier circuit is connected to the supply, to the transducer arrangement and to the ground; and
   wherein, between the transducer arrangement and the ground, a connection selector (20) is provided that is electrically non-conductive below a threshold current or threshold voltage, and electrically conductive above the threshold current or the threshold voltage.

2. Device according to claim 1, wherein the device is for intravascular measurements, the elongate body is flexible and is configured to be steered within a vascular structure of the subject, and wherein the transducer arrangement is configured to generate measuring emission in a first mode, and to receive sensing signals and to provide respective signals in a second mode.

3. Device according to claim 1 or 2, configured as a device for ultrasound imaging, wherein the transducer arrangement is an ultrasound transducer arrangement (28) configured to emit ultrasound waves, to receive ultrasound echo waves and to provide sensing signals based on the received ultrasound echo waves.

4. Device according to any of the preceding claims, wherein the connection selector is provided as a passive component with a connection selection based on a present electrical current.

5. Device according to any of the preceding claims, wherein the connection selector comprises two antiparallel diodes (30. 32);
   wherein the diodes are provided with a diode characteristic matching with a characteristic of at least one of the group of the electric energy supply for the transducer arrangement and the electric energy supply for the amplifier circuit and the amplifier characteristics; and
   wherein the voltage and/or current of the electric energy supply for the transducer arrangement is above the threshold current or threshold voltage of the diode characteristic.

6. Device according to one of the preceding claims, wherein the transducer arrangement is supplied with a unipolar or bi-polar stimulus voltage/current.

7. Device according to one of the preceding claims, wherein the ground comprises a ground conductor (34); and
   wherein the wiring arrangement is:

   i) a two-wire connection (36) comprising only the ground conductor and the supply comprises one supply conductor; or
   ii) a three-wire connection (40) comprising only the ground conductor and the supply comprises one transducer-supply conductor and one amplifier-supply conductor.

8. Device according to one of the preceding claims, wherein the transducer arrangement is connected with a first transducer-port (46) to the supply, and with a second transducer-port (48) to the ground; wherein the amplifier circuit is connected with a first amplifier-port (50) to the supply, with a second amplifier-port (52) to the ground, and with a third amplifier-port (54) to the second transducer-port; and wherein the connection selector is arranged between the second transducer-port and third amplifier-port and the ground.

9. Device according to one of the preceding claims, wherein the amplifier circuit comprises low voltage transistors, wherein resistors are arranged between the supply and the transistors.

10. A console (100) for interventional measurements, the console comprising:

    - a first electric energy supply (102) for supply of a transducer arrangement;
    - a second electric energy supply (104) for supply of an amplifier circuit;
    - a unit (106) for amplifying a received signal; and
    - an interface arrangement (108) comprising at least one supply interface (112) for connection with a device for interventional measurements according to one of the preceding claims.

11. Console according to claim 10, wherein a switching arrangement (112) is provided for alternate connection of the first electric energy supply or the amplifier to the supply interface; and
    wherein the second electric energy supply remains connected to the supply interface.

12. Console according to claim 10, wherein the interface arrangement comprises:

    - a first supply interface (116) connected to the first electric energy supply, the first supply interface for connection with a transducer supply of the device for interventional ultrasound imaging, and
    - a second supply interface (118) connected to the second electric energy supply and the unit for amplifying, the second supply interface for connection with an amplifier circuit of the device for interventional ultrasound imaging.

13. Console according to one of claims 10 to 12, wherein the transducer arrangement is supplied by a bi-polar energy supply with positive and negative voltage pulses.

14. A system (200) for interventional measurements, comprising:

    - a device (202) for interventional measurements according to one of the claims 1 to 9; and
    - a console (204) according to one of the claims 10 to 13;

    wherein, the device for interventional measurements is connectable to the console.

15. A method (300) for interventional measurements, the method comprising the following steps:

a) providing (302) an electrical stimulus voltage/current to a transducer arrangement, provided in a distal portion of an elongate body configured to be at least partly inserted into an anatomical structure of a subject, energizing the transducer arrangement;

b1) receiving (304) sensing signals by the transducer arrangement and providing respective signals to a console;

b2) providing (306) an electrical amplification supply voltage/current to an amplifier circuit provided in the distal portion of the elongate body and amplifying the signals from the transducer arrangement with the amplifier circuit; and

c) supplying (308) the amplified signals to a proximal end of the elongate body;

wherein the electrical stimulus voltage/current, the electrical amplification supply voltage/current and the amplified signals are provided by a wiring arrangement along the elongate body, the wiring arrangement comprising a ground and a supply; wherein the transducer arrangement is connected to the supply and to the ground; and the amplifier circuit is connected to the supply, to the transducer arrangement and to the ground; and wherein the transducer arrangement and the ground are selectively connected with a connection selector that is electrically non-conductive below a threshold current or threshold voltage, and electrically conductive above the threshold current or the threshold voltage.

**FIG. 1**

**FIG. 2**

**FIG. 3**

**FIG. 4**

**FIG. 5**

**FIG. 6**

102

112

s2

106

s1

110    12    16,24    14

VDD

104

**FIG. 7**

102

110

116

HVTX

RX

106

118    12

VDD    16,24    14

104

**FIG. 8**

200

204    202

**FIG. 9**

a)    302

b1)    304

b2)    306

c)    308

**FIG. 10**

Europäisches
Patentamt

European
Patent Office

Office européen
des brevets

# EUROPEAN SEARCH REPORT

Application Number

EP 19 15 4455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NATHANIEL K. COULSON ET AL: "Ultrasound stylet for non-image-guided ventricular catheterization", JOURNAL OF NEUROSURGERY. PEDIATRICS, vol. 16, no. 4, 1 October 2015 (2015-10-01), pages 393-401, XP055608937, US ISSN: 1933-0707, DOI: 10.3171/2015.2.PEDS14387 * the whole document * | 1,5-15 | INV. A61B8/12 A61B8/00 B06B1/02 G01S7/52 |
| A | Microchip: "Microchip MD0100 - Single/Dual-Channel High-Voltage Protection T/R Switch - Datasheet", Microchip Technology Inc, 1 January 2018 (2018-01-01), XP055608969, Retrieved from the Internet: URL:http://ww1.microchip.com/downloads/en/ DeviceDoc/MD0100-Single-and-Dual-Channel%2 0High-Voltage-Protection-TR-Switch-Data-Sh eet-20005738A.pdf [retrieved on 2019-07-25] * the whole document * | 1-15 | |
| X | AMIN NIKOOZADEH ET AL: "Forward-looking volumetric intracardiac imaging using a fully integrated CMUT ring array", ULTRASONICS SYMPOSIUM (IUS), 2012 IEEE INTERNATIONAL, 1 September 2009 (2009-09-01), pages 511-514, XP055608998, ISSN: 1948-5719, DOI: 10.1109/ULTSYM.2009.5441600 ISBN: 978-1-4673-4561-3 * the whole document * | 1-15 | TECHNICAL FIELDS SEARCHED (IPC) A61B B06B |

The present search report has been drawn up for all claims

| Place of search | Date of completion of the search | Examiner |
|---|---|---|
| Munich | 30 July 2019 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013030919 A1